# EUROPÄISCHE PATENTSCHRIFT

(11) **EP 0 630 226 B1**
(45) Veröffentlichungstag und Bekanntmachung des Hinweises auf die Patenterteilung: **06.12.1995**
(21) Anmeldenummer: 93905316.1
(22) Anmeldetag: 08.03.1993
(51) Int. Cl.: A61K 7/13, C09B 57/00

(54) **FÄRBEN VON KERATINISCHEN FASERN MIT INDOLINEN UNTER METALLKATALYSE**
DYEING OF KERATIN FIBRES USING INDOLINES WITH METAL CATALYSIS
COLORATION DE FIBRES KERATINIQUES AU MOYEN D'INDOLINES OBTENUES PAR CATALYSE METALLIQUE

(30) Priorität: 16.03.1992 DE 4208297
(43) Veröffentlichungstag der Anmeldung: 28.12.1994
(73) Patentinhaber: Henkel Kommanditgesellschaft auf Aktien, 40191 Düsseldorf (DE)
(72) Erfinder: KNÜBEL, Georg, D-4000 Düsseldorf 1 (DE); HÖFFKES, Horst, D-40595 Düsseldorf (DE); NEUHAUS, Winifried, D-4020 Mettmann (DE); LIESKE, Edgar, D-40213 Düsseldorf (DE)
(86) Internationale Anmeldenummer: EP9300521
(87) Internationale Veröffentlichungsnummer: WO9318738

(56) Entgegenhaltungen:
- EP-A- 0 415 802
- EP-A- 0 462 857
- GB-A- 2 132 642
- GB-A- 2 187 456

## Beschreibung

Die Erfindung betrifft Oxidationsfärbemittel zum Färben von keratinischen Fasern, insbesondere menschlichen Haaren, auf der Basis von Indolinderivaten, insbesondere 5,6-Dihydroxyindolin, wobei die oxidative Farbentwicklung durch lithium-, magnesium-, calcium-, aluminium- oder zinksalzkatalysierte Luftsauerstoffoxidation ohne den Einsatz zusätzlicher Oxidationsmittel erfolgt.

Für das Färben von keratinischen Fasern, z. B. Wolle, Pelzen oder Haaren, spielen die sogenannten Oxidationshaarfärbemittel wegen ihrer intensiven Farben und guten Echtheitseigenschaften eine bevorzugte Rolle. Solche Färbmittel enthalten Oxidationsfarbstoffvorprodukte in einem kosmetischen Träger. Als Oxidationsfarbstoffvorprodukte werden Entwicklersubstanzen und Kupplersubstanzen eingesetzt. Die Entwicklersubstanzen bilden unter dem Einfluß von Oxidationsmitteln, meist H₂O₂. untereinander oder unter Kupplung mit einer oder mehreren Kupplerkomponenten die eigentlichen Farbstoffe aus.

Als Entwicklersubstanzen werden üblicherweise Paraaminophenol- und Paraphenylendiaminderivate eingesetzt. Als sogenannte Kupplersubstanzen kommen Methaphenylendiaminderivate, Naphthole, Resorcinderivate und Pyrazolone zum Einsatz.

Färbemittel zum Färben von keratinischen Fasern müssen gute färbetechnische Eigenschaften wie z. B. gutes Aufziehvermögen auf die Faser bei möglichst niedrigen Temperaturen besitzen; die erzielte Färbung muß über gute Echtheitseigenschaften verfügen. Andererseits aber muß das Färbemittel toxikologisch und dermatologisch unbedenklich sein, vor allem wenn menschliche Haare gefärbt werden sollen.
Die in üblichen Oxidationsfärbemitteln enthaltenen Paraaminophenol- und Paraphenylendiaminderivate können jedoch bei einzelnen Personen Sensibilisierungen und Allergien hervorrufen. Es hat daher nicht an Versuchen gefehlt, Paraaminophenol- und Paraphenylendiaminderivate durch andere, wenn möglich "naturidentische" Oxidationsfarbstoffvorprodukte zu ersetzen. Hier bieten Indolderivate, die Grundbausteine des im Haar enthaltenen natürlichen Melaninfarbstoffs, eine Möglichkeit.

Viele Indolderivate kommen jedoch wegen ihrer zu hohe Oxidationsempfindlichkeit und damit verbundenen Handhabungsprobleme zur Verwendung in kommerziell verwertbaren Haarfärbemitteln nicht in Frage.

Indolinderivate, wie sie die deutsche Offenlegungsschrift DE-A 40 16 177 beschreibt, sind eine vielversprechende Alternative. Sie sind lagerstabil und bilden durch Oxidation die entsprechenden Indolderivate "in situ"; diese polymerisieren dann anschließend zum eigentlichen Farbstoff.

Als Oxidationsmittel wird üblicherweise H₂O₂ verwendet. H₂O₂ führt jedoch zu Veränderungen der ursprünglichen Eigenschaften des Haares; so ist H₂O₂-behandeltes Haar trockener, spröder und schwieriger auszukämmen: Es ist poröser und deshalb empfindlicher gegen den Einfluß von Feuchtigkeit und benötigt länger zum Trocknen, seine Wiederstandfähigkeit und Reißfestigkeit ist vermindert. Man ist deshalb bemüht, Haarfärbemittel ohne H₂O₂ oder andere Oxidationsmittel zu entwickeln und die oxidative Haarausfärbung mit Luftsauerstoff zu erreichen. Ein völliger Verzicht auf H₂O₂ führt jedoch nur zu unbefriedigenden Färbeergebnissen; so ist die erzielbare Farbintensität im Falle von Indolinderivaten als Oxidationsfarbstoffvorprodukten nur gering.

Die europäische Offenlegungsschrift EP-A 0 462 857 beschreibt u. a. Haarfärbeverfahren mit Indolinen als Oxidationsfarbstoffvorprodukten, wobei die Oxidation der Indoline bereits mit Luftsauerstoff in Gegenwart katalytischer Mengen von Metallen der 3. - 8. Nebengruppe und der Lanthanidenreihe erfolgt. Besonders bevorzugt sind Kupfersalze.

Dieses Färbeverfahren genügt jedoch nicht den hohen färbetechnischen und toxikologischen Anforderungen, die an Haarfärbemittel zu stellen sind: Zum einen sind die meisten Metallsalze selbst farbige Verbindungen, so daß eine Einflußnahme auf die erzielte Färbung nicht in jedem Falle auszuschließen ist, zum anderen stellen viele der eingesetzten Metallsalze selbst Oxidationsmittel dar. Der gravierendste Nachteil ist jedoch, daß viele der beschriebenen Schwermetallsalze toxikologisch keineswegs unbedenklich sind.

Aufgabe der vorliegenden Erfindung ist es deshalb, ein Mittel zum Färben von keratinischen Fasern, insbesondere menschlichem Haar zur Verfügung zu stellen, das Indolinderivate als Oxidationsfarbstoffvorprodukte enthält, das innerhalb kurzer Zeit kräftige Färbungen ohne den Zusatz von Oxidationsmitteln ausbildet und das als Oxidationskatalysatoren nur toxikologisch unbedenkliche, farblose Metallsalze enthält, wobei typische Schwermetallsalze wie z. B. Mangan-, Kobalt-, Kupfer- oder Silbersalze von vornherein auszuschließen sind.

Überraschenderweise wurde nun gefunden, daß auch die toxikologisch unbedenklichen redoxneutralen Hauptgruppenmetallsalze des Lithiums, Magnesiums, Calciums und Aluminiums, aber auch die Salze des aufgrund seiner abgeschlossenen d-Schale hauptgruppenmetallähnlichen Zinks die Luftoxidation von in Färbemitteln enthaltenen Indolinderivaten katalysieren.

Gegenstand der Erfindung ist daher ein Mittel zur oxidativen Färbung von keratinischen Fasern, insbesondere menschlichem Haar, welches eine Komponente A und eine Komponente B umfaßt, wobei Komponente A dadurch definiert ist, daß sie als Oxidationsfarbstoffvorprodukte Indolinderivate der Formel I 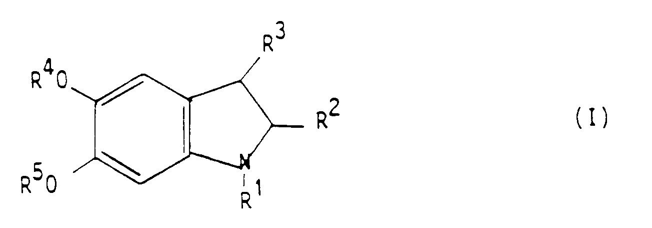
worin R¹, R², R³, R⁴ und R⁵ unabhängig voneinander Wasserstoff oder Alkylgruppen mit 1 bis 4 C-Atomen oder R⁴ und R⁵ gemeinsam mit den Sauerstoffatomen, an die sie gebunden sind, eine Alkylendioxygruppe mit 1 bis 4 C-Atomen darstellen oder deren Salze enthält, und Komponente B dadurch definiert ist, daß sie mindestens ein Metallsalz mit einem nichtoxidierend wirkenden Anion, ausgewählt aus der Gruppe der Lithium-, Magnesium-, Calcium-, Aluminium- oder Zinksalze, enthält.

Mit den erfindungsgemäßen Färbemitteln werden bereits durch Luftsauerstoffoxidation ohne den Einsatz zusätzlicher Oxidationsmittel und toxikologisch bedenklicher Schwermetallsalze besonders natürliche Farbnuancen im Bereich von Mittelbraun bis Schwarz mit guten Echtheitseigenschaften erzielt.

Die Komponenten A und B können gemeinsam in einer wasserhaltigen Zubereitung oder aber auch in zwei voneinander getrennten wasserhaltigen Zubereitungen bereitgestellt werden.

Bei Bereitstellung der Komponenten A und B in einer für die Anwendung auf dem Haar gebrauchsfertigen gemeinsamen Zubereitung ist während Herstellung und Lagerung der Zubereitung besonders auf den Ausschluß von Luftsauerstoff zu achten, um eine vorzeitige Oxidation der Farbstoffvorprodukte zu verhindern.

Bei einer getrennten Bereitstellung der Komponenten A und B werden die beiden Komponenten erst unmittelbar vor der Anwendung auf dem Haar miteinander vermischt. Es ist auch möglich, zunächst nur eine der beiden Komponenten A und B auf das Haar aufzutragen und nach einer Einwirkzeit, die zwischen einigen Sekunden und 30 Minuten beträgt, die zweite Komponente hinzuzugeben.

Eine weitere Möglichkeit besteht schließlich darin, zunächst nur eine der beiden Komponenten A und B auf das Haar aufzutragen, einwirken zu lassen, auszuspülen und erst unmittelbar nach dem Spülvorgang die zweite Komponente aufzutragen. Es spielt dabei keine Rolle, welche der beiden Komponenten A und B zuerst aufgetragen wird.
Unabhängig von der angewandten Färbetechnik wird das Haar nach dem Färbeprozeß mit einem neutral bis schwach sauer eingestellten Shampoo gewaschen.

Der in der folgenden Beschreibung gebrauchte Begriff "gesamte Färbemittelzubereitung" bezeichnet unabhängig von der Färbetechnik das gesamte, Komponente A und Komponente B umfassende Färbemittel.

Die erfindungsgemäßen Färbemittel können nicht nur zum Färben von keratinischen Fasern, sondern auch zum Färben anderer Naturfasern, wie z. B. Seide, Leinen, Baumwolle, Jute und Sisal, modifizierter Naturfasern, wie z. B. Regeneratcellulose, Nitro- und Acetylcellulose, Alkyl-, Hydroxyalkyl- und Carboxyalkylcellulose und synthetischer Fasern, wie z. B. Polyamid, Polyester-, Polyacrylnitril- und Polyurethanfasern verwendet werden.

Zur Modifikation der Farbnuancen können andere bekannte Oxidationsfarbstoffvorprodukte und gegebenenfalls auch bekannte direktziehende Farbstoffe gemeinsam mit den Indolinen der Formel I eingesetzt werden. Die Indoline der Formel I werden jedoch bevorzugt als einzige Oxidationsfarbstoffvorprodukte eingesetzt. Es ist dabei aber nicht notwendig, daß ein einheitliches Indolin der Formel I vorliegt, vielmehr kann auch ein Gemisch von verschiedenen Indolinen der Formel I zum Einsatz kommen. Die Indoline der Formel I können in freier Form oder in Form ihrer Salze, bevorzugt als Hydrochloride, Hydrobromide, Sulfate, Phosphate, Acetate, Propionate, Lactate und Citrate eingesetzt werden.

Das bevorzugt geeignete Indolin der Formel I ist 5,6-Dihydroxyindolin; dieses bildet zunächst 5,6-Dihydroxyindol und dann im Zuge einer oxidativen Polymerisation einen Melaninfarbstoff. Auch alkylsubstituierte Indoline der Formel I eignen sich als Melanin-Vorläufer, insbesondere diejenigen, bei welchen eine der Gruppen R¹, R² und R³ eine Methylgruppe ist.

Ein weiterer Gegenstand der Erfindung ist deshalb ein Mittel zur oxidativen Färbung von keratinischen Fasern, wobei in den in der Komponente A enthaltenen Indolinderivaten der Formel I R¹, R², R³, R⁴ und R⁵ Wasserstoff oder gegebenenfalls eine der Gruppen R¹, R² und R³ eine Methylgruppe ist und die übrigen Wasserstoff sind.

Zur Herstellung der Komponente A werden die Oxidationsfarbstoffvorprodukte in einen geeigneten Träger eingearbeitet. Solche Träger sind z. B. Cremes, Emulsionen, Gele oder auch tensidhaltige, schäumende Lösungen (Shampoos), Schaumaerosole oder sonstige Zubereitungen, die für die Anwendung auf dem Haar geeignet sind. Solche Träger enthalten Konfektionierungs- und Färbehilfsmittel, welche die Stabilität der Zubereitungen erhöhen und das Ergebnis der Färbung verbessern. Solche Zusätze sind in erster Linie oberflächenaktive Stoffe, z. B.
- Seifen, insbesondere die Alkali- oder Alkanolaminseifen von linearen C₁₂-C₁₈-Fettsäuren, insbesondere von Ölsäure,
- anionische Tenside, z. B. Fettalkoholsulfate und Fettalkoholpolyglycolethersulfate, Alkansulfonate, alpha-Olefinsulfonate oder Ölsäuresulfonate, bevorzugt in Form der Alkali-, Ammonium- oder Alkanolammoniumsalze,
- kationische Tenside, z. B. Alkyl(C₁₂-C₁₈-trimethyl-ammoniumchloride, Alkyl(C₁₂-C₁₈)-dimethyl-benzyl-ammoniumsalze, Cetylpyridiniumchlorid, 2-Hydroxydodecyl-hydroxyethyldimethylammoniumchlorid,
- zwitterionische Tenside, wie z. B. Alkyl-(C₁₂-C₁₈)-dimethyl-ammonium-glycinat, Kokosacylaminopropyl-dimethyl-ammonium-glycinat oder Imidazoliniumbetaine,
- amphotere Tenside, wie z. B. N-Dodecylaminoessigsäure, N-Cetylaminopropionsäure, gamma-Laurylamino-buttersäure und
- nichtionische Tenside, insbesondere Anlagerungsprodukte von 5 - 30 Mol Ethylenoxid an Fettalkohole, an Alkylphenole, an Fettsäuren, an Fettsäurealkanolamide, an Fettsäurepartialglyceride, an Fettsäure-sorbitanpartialester oder an Fettsäuremethylglucosid-partialester, ferner Alkylglucoside, Aminoxide und Fettsäure-polyglycerinester.

Weitere Konfektionierungshilfsmittel sind z. B.
- wasserlösliche, verdickende Polymere (Hydrokolloide), z. B. Celluloseether wie Carboxymethylcellulose, Hydroxyethylcellulose, Methylcellulose, Methylhydroxypropylcellulose, Stärke und Stärkeether, Pflanzengumme, Guar-Gum, Agar-Agar, Alginate, Xanthan-Gum oder synthetische wasserlösliche Polymere,
- Antioxidantien, z. B. Ascorbinsäure, Na₂SO₃,
- Puffersubstanzen, z. B. Ammoniumchlorid und Ammoniumsulfate,
- Komplexbildner, z. B. 1-Hydroxyethan-1,1-diphosphonsäure, Nitrilotriessigsäure oder Ethylendiamintetraessigsäure oder deren Salze,
- haarkosmetische Hilfsmittel, z. B. wasserlösliche kationische Polymere, Proteinderivate, Glucose, D-Panthenol, Cholesterin, Vitamine oder Pflanzenextrakte,
- Egalisierhilfsmittel, z. B. Urazol, Hexahydropyrimidin-2-on, Imidazol, 1,2,4-Triazol oder Jodide, z. B. Natrium- oder Kaliumjodid.

Die Anwendung der erfindungsgemäßen Färbemittel kann unabhängig von den oben beschriebenen Färbetechniken im pH-Bereich von 3 - 10 erfolgen, bevorzugt ist jedoch die Anwendung bei einem pH-Wert von 8,5 - 10.

Eine bevorzugte Ausführungsform der Erfindung sind solche Färbemittel, deren Komponente A Indoline der Formel I oder deren Salze in einer Menge von 0,1 - 30 Millimol pro 100 g der gesamten Färbemittelzubereitung und als Träger ein Gel mit einem Gehalt von 1 - 20 Gew.-% einer Seife oder eine Öl-in-Wasser-Emulsion mit einem Gehalt von 1 - 25 Gew.-% einer Fettkomponente und 0,5 - 30 Gew.-% eines Emulgiermittels, bezogen auf Komponente A oder die gesamte Färbemittelzubereitung, aus der Gruppe der anionischen, nichtionischen, kationischen, zwitterionischen oder ampholytischen Tenside enthalten.

Als in der Komponente B enthaltene Metallsalze kommen alle wasserlöslichen Salze des Lithiums, Magnesiums, Calciums, Aluminiums oder Zinks in Frage, die kein oxidierend wirkendes Anion, wie z. B. Nitrat, Nitrit, Jodat, Perjodat, Hypochlorit u. ä., enthalten. Geeignete Anionen sind beispielsweise Chlorid, Bromid, Sulfat, Acetat, Citrat oder Lactat.

Es ist nicht erforderlich, daß in Komponente B ein einheitliches Metallsalz vorliegt, vielmehr kann auch eine Mischung von verschiedenen Metallsalzen zum Einsatz kommen. Um eine befriedigende Ausfärbung erzielen zu können, muß jedoch eine Mindestmenge an Metallsalz verwendet werden, die bei ca. 0,02 Gew.-%, bezogen auf die gesamte Färbemittelzubereitung, liegt; der Gehalt an Metallsalz kann bis zu 2,0 Gew.-% betragen, vorzugsweise liegt er jedoch zwischen 0,05 und 0,5 Gew.-%, bezogen auf die gesamte Färbemittelzubereitung. Je nach Metallsalz entspricht dies einer Menge von 0,1 bis 50 mMol, vorzugsweise 0,3 bis 12,5 mMol, pro 100 g der gesamten Färbemittelzubereitung.

Vorzugsweise liegt die Komponente B als Öl-in-Wasser-Emulsion oder Gel vor, wobei in der oben beschriebenen Weise anstelle eines Indolins der Formel I ein Metallsalz in den Träger eingearbeitet wird. Eine Öl-in-Wasser-Emulsion enthält 1 - 25 Gew.-% Fettkomponenten und 0,5 - 30 Gew.-% eines Emulgiermittels aus der Gruppe der anionischen, nichtionischen, kationischen, zwitterionischen oder anpholytischen Tenside, ein Gel 1 - 20 Gew.-% einer Seife, jeweils bezogen auf Komponente B oder die gesamte Färbemittelzubereitung.

Die folgenden Beispiele sollen den Erfindungsgegenstand näher erläutern, ohne ihn jedoch hierauf zu beschränken.

### Beispiele

Es wurden eine Komponente A (Indolinderivat-Komponente) und eine Komponente B (Metallsalz-Komponente) folgender Zusammensetzungen hergestellt:

| | Komponente A | Komponente B |
|---|---|---|
| Fettalkohol (C₁₂-C₁₈) | 10 g | 10 g |
| Fettalkohol(C₁₂-C₁₄)-ethersulfat (2 EO) Na-salz (28 %ig) | 25 g | 25 g |
| Ascorbinsäure | 0,5 g | - |
| 5,6-Dihydroxyindolin | 9,7 mMol | - |
| Metallsalz | - | 7,5 mMol |
| konzentrierte NH₃-Lösung | bis pH = 9,0 | bis pH = 9,0 bis 9,8 |
| Wasser | ad 100 g | ad 100 g |

100 g von Komponente A und 50 g von Komponente B wurden miteinander vermischt und diese Mischung dann auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen neutral bis schwach sauer eingestellten Haarwaschmittel ausgewaschen und anschließend getrocknet.

In Abhängigkeit des verwendeten Metallsalzes resultierten brillante, farbintensive Nuancen im Braun- bis Schwarz-Bereich (Tabelle 1). Die Ausfärbungen zeichneten sich durch sehr gute Echtheitseigenschaften aus.

**Tabelle 1**

| Metallsalz | pH | erzielte Nuance | Farbintensität |
|---|---|---|---|
| LiCl | 9,0 | braunschwarz | gut |
| MgSO₄ | 9,8 | braunschwarz | gut |
| CaCl₂ | 9,0 | braun | gut |
| AlCl₃ | 9,8 | braunschwarz | gut |
| Zn-acetat | 9,0 | schwarz | sehr gut |

In einem weiteren Versuch wurde zunächst nur Komponente A auf das Haar aufgetragen, nach einer Einwirkzeit von 30 Minuten das Haar gespült und anschließend mit Komponente B in Form einer 1 Gew.-%igen wäßrigen Metallsalzlösung benetzt, nach 15 Minuten Einwirkzeit shampooniert, gespült und getrocknet.

Es wurden gleich gute Färbeergebnisse erzielt.

## Patentansprüche

1. Mittel zur oxidativen Färbung von keratinischen Fasern, insbesondere menschlichem Haar, umfassend eine Komponente A und eine Komponente B, dadurch gekennzeichnet, daß die Komponente A als Oxidationsfarbstoffvorprodukte Indolinderivate der Formel I 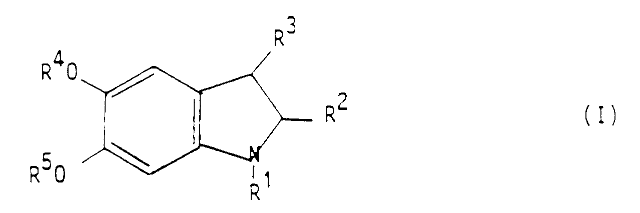 worin R¹, R², R³, R⁴ und R⁵ unabhängig voneinander Wasserstoff- oder Alkylgruppen mit 1 bis 4 C-Atomen oder R⁴ und R⁵ gemeinsam mit den Sauerstoffatomen, an die sie gebunden sind, eine Alkylendioxygruppe mit 1 bis 4 C-Atomen darstellen oder deren Salze enthält, und die Komponente B mindestens ein Metallsalz mit einem nichtoxidierend wirkenden Anion, ausgewählt aus der Gruppe der Lithium-, Magnesium-, Calcium-, Aluminium- oder Zinksalze, enthält.

2. Mittel nach Anspruch 1, dadurch gekennzeichnet, daß in den in Komponente A enthaltenen Indolinderivaten der Formel I R¹, R², R³, R⁴ und R⁵ Wasserstoff oder gegebenenfalls eine der Gruppen R¹, R² und R³ eine Methylgruppe ist und die übrigen Wasserstoff sind.

3. Mittel nach Anspruch 1 oder 2, dadurch gekennzeichnet, daß in Komponente A die Indoline der Formel I oder deren Salze in einer Menge von 0,1 bis 30 mMol pro 100 g der gesamten Färbemittelzubereitung und in Komponente B die Metallsalze in einer Menge von 0,1 bis 50 mMol, vorzugsweise 0,3 bis 12,5 mMol, pro 100 g der gesamten Färbemittelzubereitung enthalten sind.

## Claims

1. Oxidation dyes for keratin fibers, more particularly human hair, comprising a component A and a component B, characterized in that component A contains as oxidation dye precursors indoline derivatives corresponding to formula I: 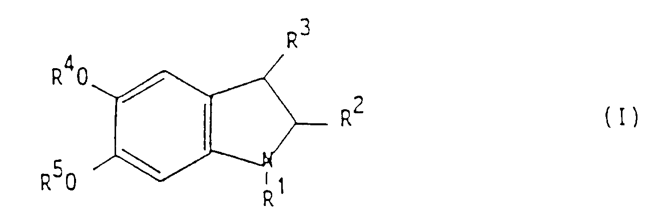 in which R¹, R², R³, R⁴ and R⁵ independently of one another represent hydrogen or alkyl groups containing 1 to 4 carbon atoms or R⁴ and R⁵ together with the oxygen atom to which they are attached represent an alkylenedioxy group containing 1 to 4 carbon atoms,
or salts thereof and in that component B contains at least one metal salt with a non-oxidizing anion selected from the group consisting of lithium, magnesium, calcium, aluminium and zinc salts.

2. Oxidation dyes as claimed in claim 1, characterized in that, in the indoline derivatives of formula I present in component A, the substituents R¹, R², R³, R⁴ and R⁵ are hydrogen or, optionally, one of these substituents R¹, R², R³ is a methyl group and the others are hydrogen.

3. Oxidation dyes as claimed in claim 1 or 2, characterized in that the indolines corresponding to formula I or salts thereof are present in component A in a quantity of 0.1 to 30 mmoles per 100 g of the dye formulation as a whole and in that the metal salts are present in component B in a quantity of 0.1 to 50 mmoles and preferably in a quantity of 0.3 to 12.5 mmoles per 100 g of the dye formulation as a whole.

## Revendications

1. Agent pour la coloration par oxydation de fibres kératiniques, en particulier des cheveux humains, comprenant un composant A et un composant B, caractérisé en ce que le composant A contient comme produits précurseurs de colorant par oxydation, des dérivés d'indoline de formule I : 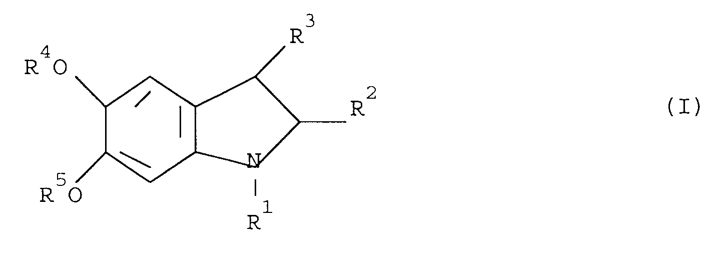 dans laquelle R¹, R², R³, R⁴ et R⁵ indépendamment l'un de l'autre, représentent de l'hydrogène ou des groupes alcoyle ayant de 1 à 4 atomes de carbone, ou R⁴ et R⁵ ensemble avec les atomes d'oxygène auxquels ils sont liés représentent un groupe alcoylènedioxy ayant de 1 à 4 atomes de carbone ou leurs sels,
et le composant B contient au moins un sel métallique avec un anion qui n'a pas d'action oxydante, choisi dans le groupe des sels de lithium, de magnésium, de calcium, d'aluminium ou de zinc.

2. Agent selon la revendication 1, caractérisé en ce que dans les dérivés d'indoline de formule I contenus dans le composant A, R¹, R², R³, R⁴ et R⁵ sont de l'hydrogène, ou éventuellement un des groupes R¹, R² et R³ est un groupe méthyle et les radicaux restants sont de l'hydrogène.

3. Agent selon la revendication 1 ou 2, caractérisé en ce que dans le composant A, les indolines de formule I ou leurs sels, sont présents en quantité allant de 0,1 à 30 mMol pour 100 g de la préparation totale de colorant et dans le composant B les sels métalliques sont présents en quantité allant de 0,1 à 50 mMol, de préférence de 0,3 à 12,5 mMol pour 100 g de la composition totale de colorant.
